# EUROPEAN PATENT SPECIFICATION

(11) **EP 0 494 996 B1**
(45) Date of publication and mention of the grant of the patent: **20.12.1995**
(21) Application number: 90916604.3
(22) Date of filing: 28.09.1990
(51) Int. Cl.: A61K 9/16

(54) **ERODIBLE MACROPOROUS HYDROGEL PARTICLES AND PREPARATION THEREOF**
ERODIERBARE MAKROPORÖSE HYDROGELPARTIKELN SOWIE DEREN HERSTELLUNG
PARTICULES D'HYDROGEL MACROPOREUSE ERODABLES ET LEUR PREPARATION

(30) Priority: 03.10.1989 US 416567
(43) Date of publication of application: 22.07.1992
(73) Proprietor: Advanced Polymer Systems, Inc., Redwood City, CA 94063 (US)
(72) Inventor: EURY, Robert, P. 691 Spruce Street, Half Moon Bay, CA 94019 (US)
(74) Representative: Harrison, David Christopher
(86) International application number: US9005564
(87) International publication number: WO9104732

(56) References cited:
- EP-A- 0 306 236
- WO-A-88/01164
- US-A- 4 690 825
- US-A- 4 806 360
- US-A- 4 818 542
- DEASY P.B. 'microencapsulation and related drug processes' 1984 , MARCEL DEKKER , NEW-YORK USA

## Description

The present invention relates generally to compositions and systems for the delivery of active substances to an aqueous environment. More particularly, the invention relates to a polymeric hydrogel delivery system and to water-erodible hydrogel particles containing a substantially continuous network of pores.

It is often desirable to deliver a drug or other chemical at a controlled rate to an aqueous environment and particularly to an animal, including human, body environment. Drug delivery systems and devices for controlled release of drugs are well known in the art. A variety of methods have been described in the literature, including physiological modification of absorption or excretion, modification of the solvent, chemical modification of the drug, adsorption of the drug on an insoluble carrier, and use of suspensions and implantation pellets. Other methods include mixing the drug with a carrier which is gradually disintegrated by the environment, e.g. body fluids, resulting in release of the drug. It is also known to disperse the drug throughout a solid matrix material through which the drug is released by diffusion, or to enclose the drug within a capsule or container having a polymeric wall or walls through which the drug can pass by diffusion. The solid matrix or wall generally retains its structural integrity over relatively long periods of time while in contact with body fluids. Such devices are often required to be implanted into and removed from the body by surgery. Additionally, because they are not biodegradible and therefore cannot be excreted by the body itself, they are not desirable for use in certain applications.

US-A-4,220,152 and 4,220,153 disclose a device for controlled release of drugs which has a wall of porous material, the pores of which contain a hydrogel which is water insoluble and through which the drugs pass from the device.

US-A-4,298,002 discloses hydrogel membranes which encapsulate biologically active tissue and which maintain their water insolubility and retention of structural integrity over long periods of time in body fluids.

D. Horák et al. (Biomaterials (1986) 7: 188) disclose spherical particles of a hydrogel which are used for endovascular occlusion of blood fluids and are not used to deliver an active ingredient.

J. Heller et al. (Biomaterials (1983) 4: 262) disclose a bioerodible hydrogel of a water-soluble unsaturated polyester copolymerized with a water-soluble monomer. This hydrogel immobilizes large macromolecules by entangling the long-chain macromolecule in the hydrogel structure as it is formed during the polymerization reaction. This can be disadvantageous because the active agent is limited to large macromolecules whose structures can become sufficiently entangled within the hydrogel complex so that they are not released until the hydrogel polymer chains cleave. In addition, use of such a preparation procedure is unsuitable for heat- and/or radiation-labile active substances which will be inactivated under the polymerization conditions. Also, the active agent can only be chosen from those which are stable with the free radical initiator used in the polymerisation reaction. Such requirements greatly limit the number of active agents which can be used. Additionally, the beads formed by this process are solid rather than macroporous.

US-A-4,690,825, WO-A-8801164 and EP-A-0 306 236 all disclose delivery vehicles having a network of pores with an active ingredient held within the pores.

Nevertheless it would still be desirable to provide delivery compositions or systems capable of providing controlled and prolonged delivery of a large variety of active substances in an aqueous environment, such as the body by water-erosion of the particles. It would also be desirable to have compositions or systems which completely degrade to relatively small water-soluble molecules which can be more readily removed from the body, preferably by the action of bodily functions themselves.

The present invention provides for a polymeric delivery system for delivery of a variety of active substances to an aqueous environment. More particularly, it is directed to a delivery system comprising erodible polymeric particles containing a substantially continuous network of pores and an impregnant comprising an active agent absorbed within the network of pores. According to a process aspect of the invention, the active agent is introduced into the pore network of the polymeric particles after the particles have been prepared.

The particles of the present invention are polymers comprised of at least one monoethylenically unsaturated water-soluble monomer cross-linked with 3-9% by weight of at least one polyethylenically unsaturated cross-linking monomers by means of ester bonds. The active substance absorbed within the particle is released from the porous structure by diffusion in a liquid environment, after which the particles are cleaved in the liquid environment by hydrolysis of the ester bonds of the cross-linkage. Cleavage of the ester bonds results in the solubilizing or erosion of the polymer into relatively small, inert, water-soluble molecules which are able to pass from the blood through the glomeruli in the kidney and excreted from the body in the urine.

The delivery systems of the present invention are useful in delivering an active substance such as a drug to an aqueous environment. This environment may be the body of an animal, including a human, or it may be an aqueous environment used by animals, e.g. aquariums, fish ponds, animal and poultry water supplies, hydroponic systems, liquid medicines, etc. The delivery system may be introduced to the animal by oral ingestion, by hypodermic needle, by drops into the eyes, by spray into the nasal passages, and the like, for example.

According to the method aspect of the present invention, the polymeric delivery system is formed by suspension radical polymerization of the monomers in an immiscible phase including a porogen. In US-A-4 818 542 porous, dry-laden particles are prepared by polymerization in the presence of the active ingredient. In contrast, in the present invention, the monomers and the porogen are first mixed together and the resulting mixture is then suspended in the immiscible phase. The immiscible phase is then agitated to form droplets of the monomer mixture, and polymerization of the monomer mixture is initiated to form the desired beads from the droplets. The desired beads are formed by providing cross-linking between the monomers. The precise dimensions and characteristics of the beads are controlled by varying process parameters such as agitation speed and nature of the porogen and/or by varying the monomers chosen for polymerization. Once the beads are formed, the porogen is extracted from the bead product, typically using solvent extraction or evaporation. The desired active substance may then be introduced into the beads, typically by contact absorption, to create the desired final product, which may then be dehydrated if desired until ready for further use, at which time it is rehydrated. In addition to allowing the incorporation of labile active agents, such a two-step preparation process allows greater control over the structure of the bead resulting from a wider choice of porogens and reaction conditions.

### DESCRIPTION OF THE DRAWINGS

Fig. 1 shows the erosion profiles (linear scale) for the beads of Examples 2, 3 and 4.

Fig. 2 shows the release profile for the beads of Examples 2, 3, 4 and 5.

Fig. 3 shows the release profile for the beads of Example 5 using different quantities of loading.

A preferred embodiment of the delivery vehicle of the present invention is a particle with a structural network formed by three-dimensional cross-linking or copolymerization which leaves random spaces or holes which collectively form the network of pores. The polymer structure or bead physically holds an impregnant comprising an active ingredient in the network of pores when the active ingredient is absorbed into the polymeric structural network. The active substance is held or trapped in the pores until the particle is exposed to an aqueous environment, at which time the active ingredient is released from the particle by diffusion from the pore network. Subsequently, the ester bonds forming the three-dimensional structure are hydrolyzed during exposure to the aqueous environment, causing the polymeric bead structure to break apart or dissolve. Small, inert, water-soluble polymeric sub-units result which can be excreted from the body.

In their most convenient form, the particles are generally spherical in shape, due to the use of suspension polymerization as a preferred method of preparation. While the microspheres may vary widely in size, those falling within the range of 5 microns to 100 microns in diameter, preferably from 10 microns to 50 microns, will provide the best results.

The pore dimensions within the spheres may also vary widely, with optimum dimensions depending on the chemical characteristics of the polymers used as well as the characteristics of the impregnant. Different systems will thus call for different optimum ranges of pore volume distribution to obtain the most desirable properties for the overall formulation. In general, however, best results are obtrained with total pore volumes ranging from 0.01 to 4.0 cc/g, preferably from 0.1 to 2.0 cc/g, more preferably from 0.3 to 1.0 cc/g; surface areas ranging from 1 to 500 m²/g, preferably from 2 to 200 m²/g; and average pore diameters ranging from 0.001 to 3.0 micron, preferably from 0.003 to 1.0 micron. Following conventional methods of measuring and expressing pore sizes, the pore diameters are calculated from the measurement of the surface area by B.E.T. nitrogen multipoint analysis and from the measurement of the pore volumes by the mercury intrusion method. The calculation is one commonly done by those skilled in the art.

The microspheres are conveniently formed by suspension polymerization in a liquid-liquid system. In general, a first solution containing monomers, a polymerization catalyst (if used), and an inert but fully miscible liquid is formed which is immiscible with a second solution. The first solution is then suspended in the second solution, which generally contains additives such as surfactants and dispersants to promote the suspension. Both the first and the second solutions are preferably non-aqueous. Once the suspension is established with discrete droplets of the desired size, usually by agitation or stirring of the suspension, polymerization is effected (typically by activating the reactants by either increased temperature or irradiation). Once polymerization is complete, the resulting beads are recovered from the suspension. The beads at this point are solid porous structures, the polymer having formed around the inert miscible liquid, thereby forming the pore network. The inert liquid has accordingly served as a porogen, or pore-forming agent, and occupies the pores of the formed beads. This porogen is then removed and replaced by the desired active substance.

Materials suitable as porogens will be substances meeting the following criteria:
1. They are either fully miscible with the monomer mixture or capable of being made fully miscible by the addition of a minor amount of solvent that is non-miscible with the second solution;
2. They are immiscible with the second solution, or at most are only slightly soluble;
3. They are inert with respect to the monomers, and stable when in contact with any polymerization catalyst used and when subjected to any conditions needed to induce polymerization (such as temperature and radiation); and
4. They are normally liquids or have melting points below the polymerization temperature. Solids can frequently be converted to liquid form by being dissolved in a solvent or by forming eutectic mixtures.

This covers a wide range of substances for use as porogens. Preferred among these are hydrocarbons, particularly inert, nonpolar organic solvents. Some of the most convenient examples are alkanes, cycloalkanes, and aromatics. Examples of such solvents are alkanes of 5 to 12 carbon atoms, straight or branched chain; cycloalkanes of 5 to 8 carbon atoms; benzene; and alkyl-substituted benzenes such as toluene and the xylenes. Other compounds which may be used as porogens are acetone and dimethylformamide. Removal of the porogen may then be effected by solvent extraction, evaporation, or similar conventional operations.

When using the above polymerization process, the steps should be performed under an inert atmosphere such as nitrogen.

A free radical initiator or polymerization catalyst may be used in the polymerization process. Such initiators may be chosen from the alkanoyl, aroyl, alkaroyl, and aralkanoyl diperoxides and monohydroperoxides, azo compounds, peroxy esters, percarbonates and other free radical-type catalysts. As examples of such catalysts there may be named benzoyl peroxide, lauryl peroxide, diacetyl peroxide, methyl ethyl ketone peroxide, diisopropylbenzene hydroperoxide, t-butylpersulfate, isopropyl percarbonate, azo-bis-(isobutyronitrile), α,α'-azodiisobutyrate, and many others. If a polymerization catalyst is used, it must be one which does not oxidize the porogen, if the latter is susceptible to oxidation.

A further advantage of using the above two-step process is that it permits the removal of unwanted species from the polymerized structures prior to incorporation of the active ingredient impregnant. Examples of unwanted species include unreacted monomers, residual catalyst, and surface active agents and/or dispersants remaining on the sphere surfaces. An additional advantage of this technique is that it permits one to select the amount and type of porogen as a means of controlling the pore characteristics of the finished bead. One is thus no longer bound by the limitations of the impregnant as it affects the structure of the bead itself. This permits partial rather than full filling of the pores with the impregnant, and further control over pore size and distribution by selection among swelling and non-swelling porogens.

Extraction of the porogen and its replacement with (i.e., impregnation of the dry bead with) the impregnant in the two-step procedure may be effected in a variety of ways, depending on the chemical nature of the porogen and its behavior in combination with that of the other species present. The beads are first recovered from the suspension by filtration, preferably using vacuum filtration apparatus. The beads are then washed with an appropriate non-aqueous solvent to remove the porogen and organic species not bound to the polymer. Once washing is complete, the solvent itself is removed by drying, preferably in a vacuum.

Once the beads are rendered dry and free of the porogen and any other unwanted organic materials, they are impregnated with the impregnant according to conventional techniques. The most convenient such technique is contact absorption. If the active ingredient is a solid, it is first dissolved in a solvent, and the resulting solution is absorbed by the beads. The solvent may either be retained in the finished product or removed by conventional means such as evaporation or extraction using a further solvent. For those solid ingredients having limited solubility in a particular solvent, high contents in the finished bead can be attained by repeated absorptions, each followed by solvent removal.

The polymerization process and the various parameters and process conditions involved in the polymerization can be selected and adjusted as a means of controlling the pore characteristics and consequently the capacity and release characteristics of the ultimate product. For example, proper selection of the cross-linking means, the amount and type of cross-linking agent, and the amount and type of porogen are means of attaining such control. Certain polymerization conditions may also be varied to such effect, including temperature, degree of radiation where used, degree of agitation and any other factors affecting the rate of the polymerization reaction. Thus, for example, under identical polymerization conditions, the porosity can be increased by increasing the calculated or theoretical cross-linking density or by increasing the porogen concentration in the solution. An increase in porosity will increase the surface area of the bead and hence the weight percent of the porogen which can be held within the bead. To decrease the particle diameter under identical polymerization conditions, the agitation or the concentration of dispersion agents in the second solution should be increased.

Cross-linking in the polymer formation is a major means of pore size control. Additionally, the cross-linking density will control the rate of release of the active agent from the particle. Monomers which may be polymerized to produce cross-linked polymer beads in accordance with the present invention include polyethylenically unsaturated monomers, i.e. those having at least two sites of unsaturation, and monoethylenically unsaturated monomers in combination with one or more polyethylenically unsaturated monomers. In the latter case, the percentage of cross-linking may be controlled by balancing the relative amounts of monoethylenically unsaturated monomer and polyethylenically unsaturated monomer. The polymer beads of the present invention will have 3% to 9% by weight cross-linking density. The percentage cross-linking is defined among those skilled in the art as the weight of polyethylenically unsaturated monomer or monomers divided by the total weight of monomer, including both polyethylenically unsaturated and monoethylenically unsaturated monomers.

In order to impart erodibility to the polymer beads of the invention, the monoethylenically unsaturated monomers forming the backbone of the polymer must be selected from those which are water-soluble or are miscible with water. This allows the polymer chain to dissolve into smaller sub-units once the ester bonds formed by the cross-linking reaction have broken apart by hydrolysis in an aqueous environment. The resulting water-soluble polymeric sub-units may then be excreted from the body, for example.

Water-soluble or miscible monoethylenically unsaturated monomers suitable for preparing polymer beads for the polymer delivery system must be capable of forming ester bonds with the cross-linking monomers. Such compounds include acrylic acid and methacrylic acid, water-soluble esters and amides of acrylic acid and methacrylic acid such as acrylamide and hydroxyethylmethacrylamide, water-soluble vinyl lactams such as N-vinylpyrrolidone, and the like.

Water-soluble polyethylenically unsaturated monomers which ordinarily act as though they have only one unsaturated group may be used as part of the monoethylenically unsaturated monomer content.

Polyethylenically unsaturated cross-linking monomers suitable for preparing the polymer beads of this invention may be either soluble or insoluble in water, but they must be capable of forming ester bonds with the monoethylenically unsaturated monomers to form a three-dimensional cross-linked structure. Such ester bonds break apart when exposed to an aqueous environment. Suitable polyethylenically unsaturated monomers may include diallyl phthalate; ethylene glycol diacrylate; bis(acrylated polyethylene glycol); ethylene glycol dimethacrylate; trimethylolpropanetrimethacrylate; polyvinyl and polyallyl esters of ethylene glycol, of glycerol, of pentaerythritol, of diethyleneglycol, of monothio- and dithio-derivatives of glycols, and of resorcinol; divinylsulfone; allyl acrylate, diallyl maleate; diallyl fumarate, diallyl succinate; diallyl carbonate; diallyl malonate; diallyl oxalate; diallyl adipate; diallyl sebacate; divinyl sebacate; diallyl tartrate; diallyl silicate; triallyl tricarballylate; triallyl aconitate; triallyl citrate; triallyl phosphate; methylenebis(acrylamide); and the like.

Once the microspheres are formed and dried, they are impregnated with the impregnant by contact absorption. As an option, the impregnant may be used in the form of a solution in a suitable solvent. Examples of such solvents are water; liquid petrolatum; ether; petroleum ether; alcohols, including methanol, ethanol, and higher alcohols; aromatics, including benzene and toluene; alkanes, including pentane, hexane, and heptane; ketones, including acetone and methyl ethyl ketone; chlorinated hydrocarbons, including chloroform, carbon tetrachloride, methylene chloride and ethylene dichloride; acetates, including ethyl acetate; and oils, including isopropyl myristate, diisopropyl adipate and mineral oil. After absorption of the solution, the solvent can be evaporated or, if desired, retained inside the pores together with the impregnant. Other formulating materials, such as carriers or adjuvants such as preservatives and antioxidants, for example, can also be present, and will be incorporated into the beads together with the impregnants and any other materials present.

Because the microspheres are macroporous, that is, they contain a network of interconnecting pores, the active ingredient may be used with any solvent with which it and the microspheres are compatible. Swelling of the hydrogel is not necessary in order to effect absorption of the active agent. This is in contrast to "normal" or prior art hydrogels, which require a solvent that swells the hydrogel structure itself in order for the active ingredient to be absorbed into the hydrogel. Additionally, the beads of the invention make it possible for a greatly increased amount of impregnant to be contained within the bead, as a result of absorbing impregnant into the network of pores as well as taking it up to swell the hydrogel structure, in contrast to normal hydrogels which can only take up an amount of impregnant that will swell the hydrogel structure.

The solvent chosen for use with the active substance can regulate to some extent the diffusion rate of the active substance from the hydrogel beads. For example, when the solvent used is water, the hydrogel structure will swell in size to a relatively great extent during absorption. Because of this swelling, there is a relatively slow diffusion rate of the absorbed active ingredient out of the beads. However, when the solvent is less polar, for example an alcohol such as ethanol, the bead hydrogel structure does not swell appreciably during absorption and there is a faster release of material.

The impregnant, whether it be pure active ingredient, a mixture of active ingredients or a solution of active ingredient, will generally comprise between 5% and 65% of the total weight of the impregnated beads, more generally up to about 40%. Prior art hydrogels generally are able to absorb only up to about 5% or less of an impregnant. When the active ingredient is a potent drug, it will generally be in the form of a dilute solution, and the weight percent of the active ingredient itself will range as low as 0.01% based on the total weight of the impregnated beads.

The active ingredient for use in the present invention may be any active ingredient which it is desired to release into an aqueous environment in a controlled manner and which is capable of being absorbed into a hydrogel bead according to the present invention. In a preferred embodiment of the invention, the active ingredient is a pharmaceutically active compound such as a drug for controlled release in an animal, including a human, body. The particle size of the beads can be controlled so that the size obtained is one that is convenient for administering the beads by syringe or catheter or in a nasal spray or in eye drops, for example. The beads may also be delivered by oral ingestion.

The beads or particles of the present invention are very soft and are non-irritating, particularly to sensitive body tissues such as the eyes or nose, when the beads take up a polar solvent such as water. When the beads are dry or take up a solvent other than water, they are relatively more rigid and have increased mechanical stability. This variety in physical characteristics makes the beads adaptable to a variety of uses. The beads are also nontoxic, non-antigenic and exhibit excellent compatibility with tissue of the living organism, which characteristics are also advantageous when the beads are utilized to administer active ingredients to an animal or human body, which is a preferred embodiment of the invention.

The following Examples are presented as being illustrative of the invention.

### Example 1

Polymer beads of acrylamide cross-linked with bisacrylated polyethylene glycol were prepared as follows.

A solution of a polymer of methyl vinyl ether and maleic anhydride (Gantrez® AN-139, available from GAF; 0.15 g) and a sorbitan oleate (Emsorb® 2505, available from Emery; 0.15 g), both utilized as suspending agents, in octane (139.57 g) was stirred and added to a 500 mL resin kettle with custom metal stirrer. A mixture of acrylamide (14.99 g), bisacrylated polyethylene glycol (Sartomer® 252, available from Sartomer International; 1.55 g) and azobis(isobutyronitrile) (AIBN; 0.30 g) was dissolved in dimethylformamide (DMF; 50.37 g) and added to the resin kettle. The mixture was stirred at 500 rpm and 30°C, under nitrogen, for 30 min., after which the speed of the stirrer was increased to 1000 rpm for 15 min. to form finite bead particles. The temperature of the water bath was then increased to 60°C and the mixture was stirred at 1000 rpm for 15.5 hours to effect polymerization. At this point, all of the solvent had blown off. The particle floc on the sides of the kettle was slightly pink. The floc was scraped back down into the kettle, and acetone (400 mL) was added. The mixture was agitated at 60°C and 1000 rpm, without a nitrogen atmosphere, for 1 hour to resuspend the floc and remove the DMF. The floc was then filtered and the beads were resuspended in acetone (250 mL) in a beaker with magnetic stirring for 1 hour to further remove any DMF. The beads were then filtered and dried under aspirator vacuum at 45°C overnight with occasional stirring to break up any lumps. The surface area, average bead diameter (by weight) and percentage cross-linking by weight of the resulting beads are given in Table I.

### Examples 2-5

Following the procedure of Example 1, beads were formed of the identical formulation except that the amount of Sartomer 252 was varied to obtain different amounts of cross-linking of the resulting beads. Additionally, in the beads of Example 2, 3 and 4, 1% by weight of 4-vinylpyridine was added as a comonomer. The surface area, average bead diameter (by weight) and percentage cross-linking by weight of the resulting beads are given in Table I below.

### Examples 6-8

Beads containing varying amounts of Sartomer 252 were prepared, otherwise conforming to the formulation in and basically following the procedures of Example 1, except that the agitation rate was reduced from 1000 rpm to 750 rpm and the stirrer used was a plastic anchor stirrer conforming closely to the size and shape of the reaction kettle. The surface area, average bead diameter (by weight) and percentage cross-linking by weight of the resulting beads are given in Table I below.

**TABLE I**

| Ex. | Sartomer (g) | 1% Vinyl-Pyridine | X-Links (wt. %) | Surface Area (m²/g) | Bead Diam. (Wt. Avg.) |
|---|---|---|---|---|---|
| 1 | 1.55 | - | 9 | 31.9 | 23.4 » |
| 2 | 1.55 | + | 3 | 19.7 | 18.5 » |
| 3 | 3.10 | + | 6 | 36.4 | 64.7 » |
| 4* | 6.20 | + | 12 | 84.3 | 158.3 » |
| 5 | 4.65 | - | 9 | 30.5 | 27.2 » |
| 6 | 4.65 | - | 9 | 33.4 | 17.9 » |
| 7 | 2.32 | - | 5 | 5.8 | 7.8 » |
| 8* | 9.30 | - | 18 | 3.4 | 7.3 » |

| | | | | | |
|---|---|---|---|---|---|
| * not embodying the invention. | | | | | |

### Example 9

To study the erosion capabilities of the beads of the invention, the dried beads of Example 2 were suspended in 50 mL of isopropanol, and this suspension was shaken and sonicated. Then 1-mL aliquots were added to 35 mL of phosphate buffer, pH 7.4. These aliquots were incubated at 40°C and 100 oscillations/min. At periodic intervals, the tubes were removed, the beads were separated by centrifugation at 1500 rpm for 5 min., and 5-mL aliquots were removed from the supernatant. The volume was restored with fresh phosphate buffer. The amount of eroded (solubilized) polymer was determined by UV/Vis measurements at 256 nm (the absorbance of 4-vinylpyridine). The concentration was corrected for the succeeding dilutions.

The above procedure was repeated with dried beads of Example 3 and dried beads of Example 4.

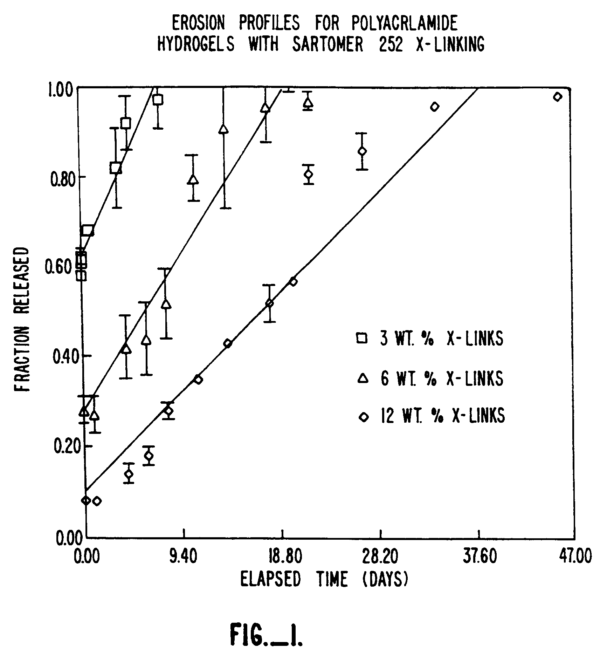

The resulting erosion profile for the beads is shown in Fig. 1.

The results show that a higher cross-link density resulted in a material with a decreased soluble fraction and a slower erosion rate. The time necessary for complete erosion was from approximately 8 days (3% cross-linking) to approximately 40 days (12% cross-linking). The erosion rates were relatively linear over time. An unexpected result was the relatively quick erosion of the 3% cross-linked beads, with a large portion of the polymer being lost within 1 hour. This is most likely the result of solubility of the water-soluble monomers because the amount of cross-linking was low. The erosion due to hydrolysis is believed to begin at about 11 hours, with the erosion being fairly linear until all the ester bonds have been cleaved (linear least squares of all points gave corr.=0.969).

### Example 10

Release rates of the beads of the present invention with different cross-linkings were evaluated as follows.

Samples of beads from Examples 2, 3, 4 and 5 were loaded with approximately 10 weight percent of D & C Red #28 dye by dissolving 0.5 g D & C Red #28 (Pyla Certified, Pylam Products) in 7.5 g of absolute ethanol. This dye was then poured as evenly as possible over 4.5 g of the sample of polymer beads to be tested in a petri dish. The material was stirred until uniform and the solvent was then evaporated. Periodic mixing during evaporation was required to even drying. When no further change was noticed (as evidenced by a lightening of the color intensity of the dyed beads), the material were dried for 4 hours under vacuum at 40°C.

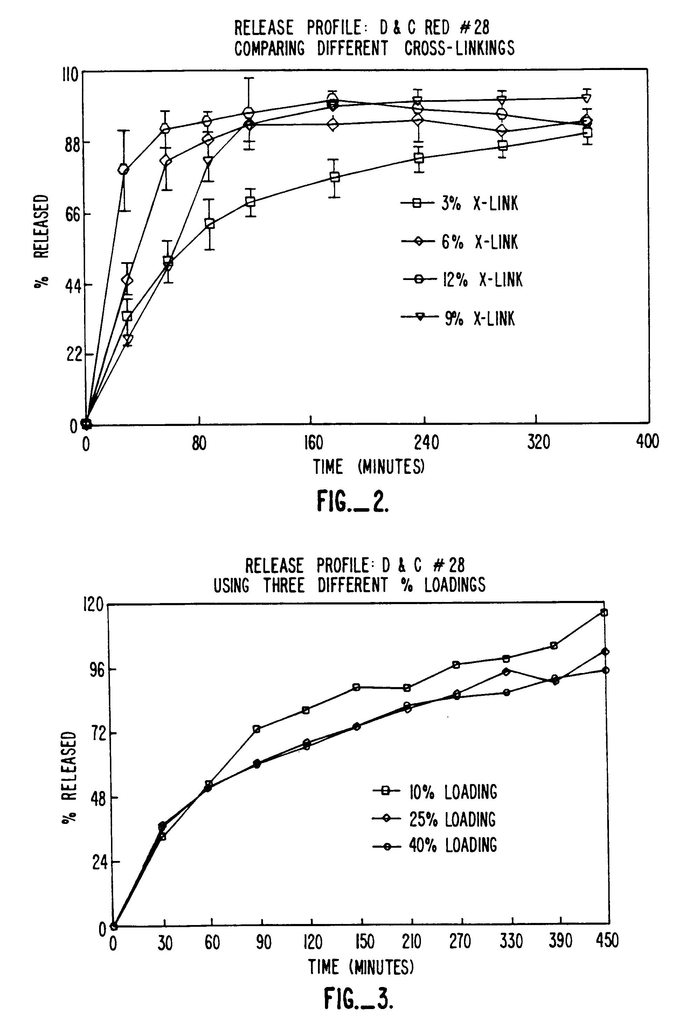

Approximately 100 mg of the sample was put into six individual baskets of a modified USP Dissolution Apparatus. The baskets were spun at 240 rpm while being lowered into 500 mL of distilled water (the dissolution medium). Once the baskets were in the distilled water, the speed was immediately turned down to 150 rpm. The dissolution medium was held at a constant temperature of 22°C and kept covered throughout the experiment. The dissolution medium was assayed at predetermined time intervals. Analysis of the medium was performed using a Beckman DU-65 Spectrophotometer at 538 nm. Each sample was run in duplicate. The results are shown in Fig. 2.

### Example 11

Examination of the amount of variance in the release profiles for beads loaded with different weight percentages of material was carried out as follows.

Following the procedure of Example 10, three samples of the beads of Example 5 were loaded with approximately 10%, 25% and 40%, respectively, of D & C Red #28 dye. The release rate of the dye from each sample was then determined, following the procedures of Example 10, and the results are presented in Fig. 3. The results indicate that the percent release per minute of dye from the polymer beads did not seem to be dependent on the percent loading of the polymer.

## Claims

1. A delivery vehicle for controlled release of an active agent to an aqueous environment, said vehicle comprising preformed porous, water-erodible polymeric particle having a network of pores with an impregnant comprising the active agent absorbed within the network of pores and the polymeric particle comprising a water-soluble monoethylenically unsaturated monomer cross-linked with from 3-9% by weight of a polyethylenically unsaturated monomer, the cross-linking reaction forming ester bonds.

2. A delivery vehicle according to claim 1 wherein the particle has a diameter from 5 microns to 100 microns, and a surface area in the range from 1 m²/g to 500 m²/g.

3. A delivery vehicle according to claim 1 wherein the particle is copolymerized from a monomer selected from acrylamide and N-vinylpyrrolidone and from a cross-linking monomer selected from methylenebisacrylamide and bisacrylated polyethylene glycol.

4. A delivery vehicle according to claim 1 wherein the amount of cross-linking monomer is 3-6% by weight.

5. A process for preparing a delivery vehicle for controlled release of an active agent to an aqueous environment, said process comprising the steps of:
dissolving at least one water-soluble monoethylenically unsaturated monomer and 3 to 9% by weight of a polyethylenically unsaturated cross-linking monomer, capable of forming ester linkages, in an inert porogen to form a first solution;
suspending the first solution in a second solution incompatible with the first solution;
agitating the first and second solutions to form a plurality of droplets of the first solution suspended in the second solution;
activating the monomers in the plurality of droplets to copolymerize the monomers and form a water-erodible porous particle having a network of pores with the porogen held within the network of pores;
separating the water-erodible porous particle from the second solution;
removing the porogen and any impurity from the water-erodible porous particle;
placing the water-erodible porous particle in a solution of impregnant; and
absorbing the impregnant into the network of pores of the water-erodible porous particle.

6. A process according to claim 4 wherein a comonomer pair is copolymerized from a monomer selected from acrylamide and N-vinylpyrrolidone and from a cross-linking monomer selected from methylenebisacrylamide and bisacrylated polyethylene glycol.

7. A process according to claim 4 wherein the second solution is a non-aqueous solution.

8. A process according to claim 4 wherein the amount of ester linkage forming monomer is 3 to 6% by weight.

## Patentansprüche

1. Abgabevehikel zur gesteuerten Freisetzung einer Wirksubstanz an eine wäßrige Umgebung, wobei das Vehikel vorgeformte poröse, wassererodierbare Polymerteilchen umfaßt, die ein Porennetzwerk aufweisen, in dem ein die Wirksubstanz aufweisendes Imprägniermittel absorbiert ist, und die Polymerteilchen ein wasserlösliches monoethylenisch ungesättigtes Monomer umfassen, das mit 3-9 Gew.-% eines polyethylenisch ungesättigten Monomers vernetzt ist, wobei die Vernetzungreaktion Esterbindungen bildet.

2. Abgabevehikel nach Anspruch 1, worin die Teilchen einen Durchmesser von 5 »m bis 100 »m und eine Oberfläche im Bereich von 1 m²/g bis 500 m²/g aufweisen.

3. Abgabevehikel nach Anspruch 1, worin die Teilchen aus einem aus Acrylamid und N-Vinylpyrrolidon ausgewählten Monomer und einem aus Methylenbisacrylamid und bisacryliertem Polyethylenglykol ausgewählten vernetzenden Monomer copolymerisiert sind.

4. Abgabevehikel nach Anspruch 1, worin die Menge an vernetzendem Monomer 3-6 Gew.-% beträgt.

5. Verfahren zur Herstellung eines Abgabevehikels zur gesteuerten Freisetzung einer, Wirksubstanz an eine wäßrige Umgebung, das folgende Schritte umfaßt:
das Auflösen zumindest eines wasserlöslichen, monoethylenisch ungesättigten Monomers und 3 bis 9 Gew.-% eines polyethylenisch ungesättigten, vernetzenden Monomers, das zur Bildung von Esterbindungen fähig ist, in einem inerten Porogen, um eine erste Lösung zu bilden;
das Suspendieren der ersten Lösung in einer zweiten Lösung, die mit der ersten Lösung inkompatibel ist;
das Rühren der ersten und zweiten Lösungen, um eine Vielzahl von Tröpfchen der ersten Lösung in der zweiten Lösung suspendiert zu bilden;
das Aktivieren der Monomere in der Vielzahl von Tröpfchen, um die Monomeren zu kopolymerisieren und wassererodierbare poröse Teilchen mit einem Porennetzwerk zu bilden, wobei das Porogen im Porennetzwerk gehalten wird;
das Abtrennen der wassererodierbaren porösen Teilchen aus der zweiten Lösung;
das Entfernen des Porogens und jeglicher Verunreinigung aus den wassererodierbaren porösen Teilchen;
das Anordnen der wassererodierbaren porösen Teilchen in einer Imprägniermittellösung; und
das Absorbieren des Imprägniermittels im Porennetzwerk der wassererodierbaren porösen Teilchen.

6. Verfahren nach Anspruch 5, worin ein Comonomerenpaar aus einem aus Acrylamid und N-Vinylpyrrolidon ausgewählten Monomer und einem aus Methylenbisacrylamid und bisacryliertem Polyethylenglykol ausgewählten vernetzenden Monomer copolymerisiert wird.

7. Verfahren nach Anspruch 5, worin die zweite Lösung eine nichtwäßrige Lösung ist.

8. Verfahren nach Anspruch 5, worin die Menge an Esterbindung bildendem Monomer 3 bis 6 Gew.-% beträgt.

## Revendications

1. Véhicule de délivrance pour une libération contrôlée d'un agent actif à un environnement aqueux, ledit véhicule comprenant une particule polymérique érodable par l'eau, poreuse, préformée ayant un réseau de pores avec un imprégnant comprenant l'agent actif absorbé dans le réseau de pores et la particule polymérique comprenant un monomère insaturé monoéthyliniquement soluble dans l'eau et réticulé avec de 3 à 9% en poids d'un monomère insaturé polyéthyléniquement, la réaction de réticulation formant des liaisons ester.

2. Véhicule de délivrance selon la revendication 1 dans lequel la particule a un diamètre de 5 microns à 100 microns, et une surface spécifique dans l'intervalle de 1 m²/g à 500 m²/g.

3. Véhicule de délivrance selon la revendication 1, dans lequel la particule est copolymérisée à partir d'un monomère choisi parmi l'acrylamide et la N-vinylpyrrolidone et d'un monomère de réticulation choisi parmi un méthylènebisacrylamide et un polyéthylène glycol bisacrylaté.

4. Véhicule de délivrance selon la revendication 1 dans lequel la quantité de monomère de réticulation est de 3 à 6% en poids.

5. Procédé pour préparer un véhicule de délivrance pour libération contrôlée d'un agent actif à un environnement aqueux, ledit procédé comprenant les étapes de :
dissolution d'au moins un monomère insaturé ou monoéthyléniquement soluble dans l'eau et de 3 à 9% en poids d'un monomère de réticulation insaturé polyéthyléniquement, capable de former des pontages ester, dans un porogène inerte pour former une première solution;
mise en suspension de la première solution dans une seconde solution incompatible avec la première solution;
agitation des première et seconde solutions pour former une pluralité de gouttelettes de la première solution suspendues dans la seconde solution;
activation des monomères dans la pluralité des gouttelettes pour copolymériser les monomères et former une particule poreuse érodable par l'eau ayant un réseau de pores avec le porogène maintenu dans le réseau de pores;
séparation de la particule poreuse érodable par l'eau de la seconde solution;
élimination du porogène et de toute impureté de la particule poreuse érodable par l'eau;
placement de la particule poreuse érodable par l'eau dans une solution d'imprégnant; et
absorption de l'imprégnant dans le réseau de pores de la particule poreuse érodable par l'eau.

6. Procédé selon la revendication 4, dans lequel une paire de comonomères est copolymérisée à partir d'un monomère choisi parmi l'acrylamide et la N-vinylpyrrolidone et d'un monomère de réticulation choisi parmi le méthylènebisacrylamide et le polyéthylène glycol bisacrylaté.

7. Procédé selon la revendication 4, dans lequel la seconde solution est une solution non aqueuse.

8. Procédé selon la revendication 4, dans lequel la quantité de monomère formant des pontages ester est de 3 à 6% en poids.
